(19) Europäisches Patentamt

European Patent Office

Office européen des brevets

(11) **EP 4 751 769 A1**

(12) **EUROPEAN PATENT APPLICATION**

(43) Date of publication:
03.06.2026 Bulletin 2026/23

(21) Application number: 24215917.6

(22) Date of filing: 27.11.2024

(51) International Patent Classification (IPC):
*A61N 5/10* *(2006.01)*

(52) Cooperative Patent Classification (CPC):
**A61N 5/1038;** A61N 5/1031

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Elekta Limited**
**Crawley, West Sussex RH10 9BL (GB)**

(72) Inventor: **DALFSEN, Raymond**
**Crawley, RH10 9RR (GB)**

(74) Representative: **Hamer, Thomas Daniel et al**
**Kilburn & Strode LLP**
**Lacon London**
**84 Theobalds Road**
**London WC1X 8NL (GB)**

(54) **METHOD FOR ADAPTIVE RADIOTHERAPY**

(57) Disclosed herein is a computer-implemented method for adaptive radiotherapy. The method comprises receiving symptom data, the symptom data being indicative of the existence and/or severity of a symptom experienced by a radiotherapy patient undergoing treatment according to a first radiotherapy treatment plan; obtaining first treatment plan data associated with the first radiotherapy treatment plan; and generating a second radiotherapy treatment plan based on the obtained first treatment plan data and the symptom data.

FIG. 1A

150

Fluence Map
Optimisation

151

Determine
configuration

153

FIG. 1B

**Description**

This disclosure relates to a method for adaptive radiotherapy.

**Background**

Radiotherapy can be described as the use of ionising radiation, such as X-rays, to treat a human or animal body. Radiotherapy is commonly used to treat cancer, for example to treat tumours within the body of a patient or subject. In such treatments, ionising radiation is used to irradiate, and thus destroy or damage, cells which form part of the tumour.

Adaptive radiotherapy (ART) is a way of modifying or adapting the radiotherapy during the course of treatment, for example to account for changes in the patient's anatomy or tumor characteristics. In general, frequent imaging is used to monitor changes in the tumour's shape, size, and position, as well as any similar changes in surrounding organs and tissues. ART techniques generally fall into three categories: offline, online, and real-time. Offline ART involves updating a treatment plan between fractions (e.g. between treatment days). Online ART involves updating the plan on the day of treatment, e.g. immediately before radiation is delivered. Real-time ART involves adjustment of the plan to account for motion and changes in the patient's anatomy, such as tumor position, in 'real-time' during the radiation session using real-time imaging techniques.

A downside of radiotherapy is that in addition to damaging a tumour, treatment can also damage healthy tissues and organs. In turn, this can lead to the patient experiencing side effects, such as skin reactions, fatigue, diarrhoea, and mouth and throat problems such as dry mouth and difficulty swallowing. Currently, these side effects are treated via administration of drugs, such as anti-nausea medication to treat nausea, steroids to reduce inflammation and swelling in affected tissues, and antidiarrheal medication to help manage diarrhoea. In addition, certain side effects can be managed via dietary adjustments, for example to help address loss of appetite, nausea, and gastrointestinal issues.

To assist clinicians in monitoring side effects / symptoms and in prescribing supportive treatments, patients may meet with their clinician regularly, for example weekly, to report their symptoms. This clinician may or may not be the person responsible for the patient's radiotherapy treatment plan, potentially leading to a disconnect between the clinician responsible for helping the patient manage their side effects, and the clinician responsible for modifying and updating the patient's treatment as part of an ART workflow. In addition, there is a time and cost burden associated with the current approaches to managing side effects, e.g. through medications, the need to schedule appointments and administer tests, and so on.

The present invention seeks to address these and other disadvantages encountered in the prior art by providing an improved method for adaptive radiotherapy.

**Summary**

An invention is defined in the accompanying independent claims. Optional features are set out in the dependent claims.

**Figures**

Specific embodiments are now described, by way of example only, with reference to the drawings, in which:

Figures 1a and 1b depict optimisation procedures according to the present disclosure;
Figure 2 depicts a method according to the present disclosure;
Figure 3 depicts a method according to the present disclosure;
Figure 4a depicts a dose distribution according to an initial, 'first', or 'reference' radiotherapy treatment plan, and figures 4b and 4c depict updated dose distributions according to adapted radiotherapy treatment plans.
Figures 5a, b and c are graphs depicting changes in dose to particular anatomical tissues based on the initial radiotherapy treatment plan and the updated plans described with respect to figures 4a-c.
Figure 6 depicts a radiotherapy device according to the present disclosure.
Figure 7 depicts a system according to the present disclosure;
Figure 8 depicts a computer-readable medium according to the present disclosure.

**Detailed Description**

In overview, the application relates to a method for adaptive radiotherapy which takes into account one or more symptoms experienced by the patient when adapting a treatment plan, with the aim of limiting radiation dose to an associated anatomical region in a way which alleviates the patient's symptoms (e.g. side effects) while not compromising the therapeutic aims of the patient's course of radiotherapy.

The method comprises receiving symptom data which is indicative of the existence and/or severity of a symptom experienced by the patient. This symptom data might be self-reported, for example produced from regular questionnaires which the patient can fill in via their mobile phone. Treatment plan data is then obtained which is associated with the patient's radiotherapy treatment plan, and a new radiotherapy treatment plan is generated based on the obtained treatment plan data and the symptom data.

To date, symptoms experienced by radiotherapy patients have been managed separately from ART techniques, for example by administration of drugs or dietary changes. By taking into account the patient's symptoms in the manner described herein when adapting a treatment plan, the resulting adapted plan can play a role in mitigating the patient's symptoms (e.g. side effects) without compromising the therapeutics aims of the treatment plan.

Disclosed herein is a method for adaptive radiotherapy, comprising receiving symptom data, the symptom data being indicative of the existence and/or severity of a symptom experienced by a radiotherapy patient undergoing treatment according to a first radiotherapy treatment plan; obtaining first treatment plan data associated with the first radiotherapy treatment plan; and generating a second radiotherapy treatment plan based on the obtained first treatment plan data and the symptom data.

By generating the second radiotherapy treatment plan based at least in part on symptom data, the second radiotherapy treatment plan takes into account the patient's symptoms. For example, the dose delivered to organs causing those symptoms can be reduced in future treatment sessions in order to mitigate those symptoms.

Optionally, the method further comprises determining, based on the symptom data, an anatomical structure associated with the symptom.

Optionally, the first treatment plan data comprises a radiotherapy planning image of the patient, wherein the radiotherapy planning image depicts a target region and the anatomical structure.

Optionally, the second radiotherapy treatment plan defines a reduced dose to the anatomical structure compared with a first planned dose defined by the first treatment plan.

Optionally, the first treatment plan data comprises a first set of objectives used to guide a first optimisation process during generation of the first treatment plan.

Optionally, each of the first set of objectives has an ascribed priority and the first set of objectives is ranked in order of importance according to the ascribed priorities to provide a guide for the first optimisation process.

Optionally, the first radiotherapy treatment plan is, or has been, generated, at least in part, by sequentially optimising for the first set of objectives in turn, according to their ascribed priorities.

Optionally, generating the second radiotherapy treatment plan comprises adapting the first set of objectives to form an adapted set of objectives; and performing a second optimisation process based on the adapted set of objectives.

Optionally, adapting the first set of objectives comprises adding a new objective to the first set of objectives, the new objective relating to the anatomical structure.

Optionally, at least one of the objectives of the first set of objectives relates to the anatomical structure; and wherein adapting the first set of objectives comprises adapting the at least one objective relating to the anatomical structure to form the adapted set of objectives.

Optionally, adapting the at least one objective comprises adjusting an ascribed priority, a type, or a goal of the at least one objective.

Optionally, adapting the at least one objective comprises increasing its ascribed priority.

Optionally, each objective of the adapted set of objectives has an ascribed priority and the adapted set of objectives is ranked in order of importance according to the ascribed priorities to provide a guide for the second optimisation process, wherein the at least one objective associated with the anatomical structure has an increased priority in the adapted set of objectives in comparison with its priority in the first set of objectives.

Optionally, the first and second optimisation processes are multi-criterial optimisation processes.

Optionally, the second radiotherapy plan is a plurality of radiotherapy treatment plans, each plan of the plurality of radiotherapy treatment plans comprising a different trade-off between the objectives of the adapted set of objectives.

Optionally, both the first and the second optimisation processes are further performed based on a set of fixed constraints.

Optionally, the method further comprises determining whether the existence and/or severity of the symptom is expected or unexpected based at least in part on a stage of the first radiotherapy treatment plan, and proceeding to generate the second radiotherapy treatment plan if the existence and/or severity of the symptom is unexpected.

Optionally, the symptom data comprises, or is based on, self-reported symptom data reported by the patient.

Optionally, the generated radiotherapy treatment plan defines the delivery of therapeutic radiation for at least a next fraction of the patient's treatment.

Disclosed herein is a computer readable medium comprising instructions which, when executed by one or more processors, cause the one or more processors to implement a method according to any of the methods disclosed herein.

Disclosed herein is a radiotherapy system comprising one or more processors configured to perform according to any of the methods disclosed herein.

Optionally, the radiotherapy system further comprises a source of therapeutic radiation configured to deliver or otherwise implement the generated second radiotherapy treatment plan.

Generating a radiotherapy treatment plan is a complex process. One or more 3D images of the patient are acquired, for example a computed tomography (CT) image, cone-beam computed tomography (CBCT) image, and/or a magnetic resonance (MR) image. Anatomical structures are identified in the image(s), either by an automated segmentation tool or by a clinician. As part of this process, not only is the tumour identified in the image(s), but also any organs-at-risk (OARs). OARs are healthy organs and tissues that are sensitive to radiation and are located near the treatment area. An aim of treatment planning is to ensure that the tumour receives a prescribed level of radiation, but also to ensure that the radiation dose to any OARs is minimised in order to reduce the risk of side effects and complications. Common examples of OARs in radiotherapy include the spinal cord, heart, lungs, and kidneys, depending on the location of the tumor being treated.

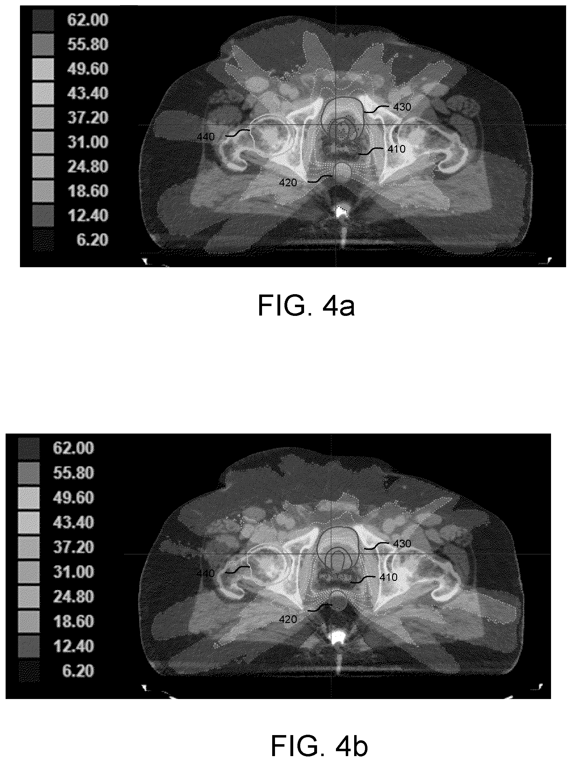

Optimisation techniques are widely used in radiotherapy treatment planning. Optimising algorithms (optimisers) are used to generate a plan which complies with various treatment constraints and/or objectives in order to produce a treatment plan that is clinically acceptable. At a high level, one or more optimisation techniques are used to convert these treatment objectives and constraints into dose distribution data. The dose distribution data describes the optimised spatial distribution of dose in the patient's anatomy, and may take the form of a 3D dose distribution map or a set of 2D dose distribution maps, for example. The dose distribution maps depict the optimised distribution of dose in the patient's body. Examples of 2D dose distribution maps are shown in figures 4a-c. One or more additional optimisation processes are then used to convert the intended dose distribution data into radiation delivery variables which can be used to deliver the treatment. Together, the radiation delivery variables may be referred to as a configuration of the radiotherapy system. Examples of radiation delivery variables include: a weight of a particular beam, the beam energy level and dose rate, the beam shape (e.g. defined by positions of multi-leaf collimator leaves for a particular beam); gantry angle at which radiation is to be delivered, beam on and off times, and the like. A treatment plan may define a plurality of control points, with each control point having specific values of these parameters.

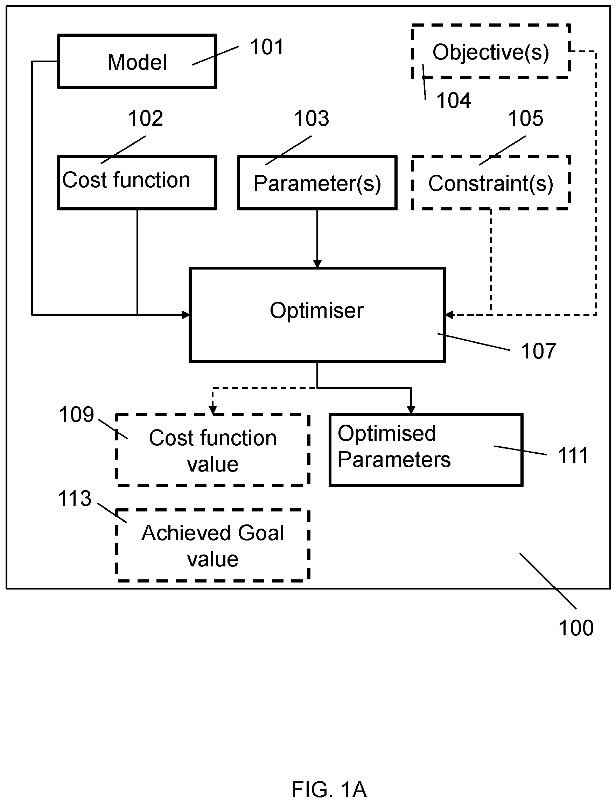

Figure 1a depicts a block diagram of an optimisation procedure 100. The optimisation procedure 100 is applicable to the optimisation of a set of parameters (i.e., one or more parameters) for a radiotherapy system, such as a system incorporating device 600 described below. The one or more parameters may be referred to as optimisable parameters, or as decision variables. Examples of optimisable parameters comprise parameters that relate to characteristics of radiation to be delivered by the radiotherapy system. For example, the set of parameters may relate to a fluence map. The fluence map may also be referred to as an intensity profile. The fluence map may correspond to the weight of the beamlets (beamlets are described below).

The optimisation module 100 comprises an optimiser 107. Given a model 101 and one or more parameters 103, the purpose of the optimiser 107 is to provide a set of optimised parameters 111 that minimise a cost function 102. The optimiser 107 comprises at least one optimization algorithm such as a simplex algorithm, a gradient-based algorithm, and an interior point algorithm, etc, and a combination thereof. Other optimisation algorithms are also possible. An optimisation algorithm may be executed until a stopping criterion is reached. For example, a stopping condition may be any one or more of: a number of iterations being reached, a convergence criterion being met, and a constraint (such as constraint 105 described herein) being violated. A constraint may be a predefined constraint, defined in advance of the optimisation procedure being performed. Alternatively, a constraint may be obtained from a previous optimisation procedure. Examples of optimization are provided in Monaco" Training Guide, Document ID: LTGMON0530 (Elekta AB). The optimisation procedure may, for example, correspond to the optimisation procedure described in European patent application publication EP38100A1.

The model 101 is a representation of the physical problem. For radiotherapy treatment planning, the model 101 may comprise a segmented image of the patient and may include, for example, a dose distribution over voxels in a region. The model may be or comprise a medical image segmented into several subsets of voxels, each with their own electron density values. The dose distribution may be the dose at unit fluence. The anatomical structure may comprise one or more of: a target, a region of healthy tissue (known as organs at risk or OARs), planned target volume (PTV), critical structures, or shell structures. Therefore the model 101 may comprise a radiotherapy planning image depicting at least one target region and at least one anatomical structure taking the form of an organ at risk (OAR). These structures are determined and/or defined during a process known as segmentation.

Shell structures are structures generated to tune the dose delivered to the tissue surrounding the target, and they may be used to control dose conformality. The dose distribution may be determined by pencil beam algorithms, convolution based algorithms and/or Monte Carlo (MC) based algorithms. Further details of how the dose may be determined are provided in Monaco® Training Guide, Document ID: LTGMON0530 (Elekta AB).

The one or more parameters 103 are optimisable parameters for which the optimiser 107 attempts to find optimum values. The one or more parameters 103 may be initialised to a predetermined value. In an example, for intensity-modulated radiation therapy (IMRT) and when the parameters 103 are the weight of beamlets, the parameters 103 may be initialised to a predetermined weight. Alternatively, the parameters 103 may be initialised to values determined in a previous step. For example, when a further optimisation is performed, the parameters may be initialised to values determined in a previous optimisation.

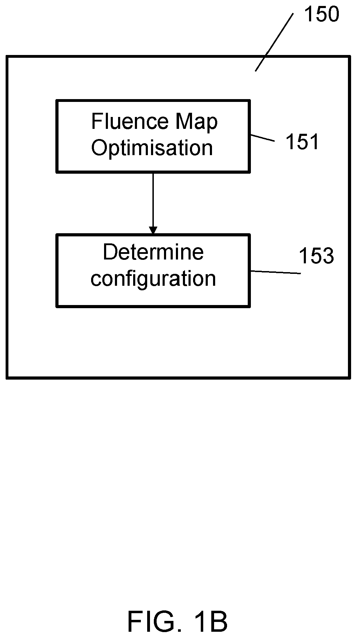

A beam may be divided into beamlets where the weight of each beamlet may be adjusted. A beam may be understood as a matrix of beamlets. Thus, a beam may be represented by a plurality of beamlets. The total dose distribution is then the weighted sum of the contribution of each beamlet at unit fluence (For example, the dose at a voxel k is given by $\Sigma x_n \cdot d_{n,k}$, where n is an index for beamlet n, $x_n$ is the weight of beamlet n, and $d_{n,k}$ is the dose at unit fluence at voxel k due to beamlet n). By tuning the weight of the beamlets, the radiation may be modulated. The weight of the beamlets may be adjusted by adjusting the configuration of a beam shaping apparatus, such as collimator 108 in figure 1. For example, when the collimator is a beam shaping apparatus such as an MLC, the optimised beamlet weights (and hence the desired dose distribution) may be implemented by using different aperture shapes of the MLC (also referred to as segments) to shape the delivered radiation. The weight of a beamlet is related to the dose rate. The dose rate refers to how much dose a linac or energy source may deliver per unit time. For a fixed dose rate, the beamlet weight is proportional to the amount of time the beamlet is active. The beamlet may be active, for example by having an opening in the beam shaping apparatus, at a location corresponding to the beamlet, such that radiation may pass through. For example, a higher weight means the beamlet is kept active for a longer time. Thus, the weight of a beamlet is a function of dose rate and/or the duration for which the beamlet is kept active.

The cost function 102 is a mathematical formulation that relates the parameters 103 and the model 101. The cost function 102 may be referred to as an objective function. The cost function 102 relates the dose distribution in the model 101 to a single value (the cost function value 109). For example, when the reference objective is "the mean dose for the bladder is less than 30 Gy", the cost function mathematically formulates and computes the mean dose for the bladder as a function of parameters 103, and compares the achieved mean dose to the reference of 30 Gy. During optimisation, the optimiser 107 seeks parameters that would minimize the difference between the achieved mean dose and the reference mean dose. The cost function value 109 quantifies how close the dose in the model is to a desired value.

The cost function value 109 is value of the cost function 102 when evaluated with the set of optimised parameters. The cost function value 109 is calculated by the optimiser during optimisation. The cost function value 109 may be referred to as a penalty. Optionally, the cost function value 109 is output by the optimiser. The cost function value 109 represents a penalty for violating an objective or constraint. The penalty is evaluated by the optimiser during optimisation. Optionally, in constrained optimisation, only objectives contribute to the cost function.

The constraint(s) 105 comprise one or more conditions that the optimised parameters 111 must satisfy. The constraint may be a hard constraint (which set conditions that the parameters are required to satisfy) or soft constraint (which have some variable values that are penalised in the cost function if some conditions are not satisfied). Constraints restrict the set of solutions that are obtainable. Constraints are used to define what is physically or clinically acceptable rather than what is mathematically possible. For example, for IMRT, a constraint may be that the weights of the beamlets must be non-negative (since a negative beamlet weight is not possible). Note that the constraint 105 is an optional feature.

The objective(s) 104 represents one or more goals to be achieved. In an example, for IMRT, the reference objective may comprise a dose-based objective and/or a volume-based objective. A dose objective may be defined in terms of a dose value (in Gy). An example of a dose objective may be stated as "the average dose to an OAR should be less than or equal to 30Gy". A volume-based objective may comprise a relative volume or an absolute volume. The relative volume represents a fraction of a volume. For example, the relative volume is a percentage. The absolute volume may be defined in terms of physical dimensions (e.g., in mm, cm, mm$^3$, cm$^3$, etc). An example of a volume based objective may be stated as "at least 90% of the volume of a PTV should meet some criterion". In another example, a reference objective 104 may be stated as "at least X% of a first region receives at least Y Gy". The optimiser 107 may then determine optimised parameters 111 that result in an achieved goal value 113 that is close to the reference objective 104. The reference objective may be referred to as a treatment-planning objective. Additionally and optionally, the reference objective 104 comprises an indication of a cost function 102 to be used. Yet optionally, the reference objective comprises an indication of an anatomical structure to which the goal to be achieved is applicable.

The reference objective is an anatomy specific function that establishes the dose and/or biological response goal. Constraints 105 are typically anatomy-specific functions that must be met. They may be referred to as hard constraints. When constraints are used together with objectives, constraints are always met, while objectives may not be met (instead, they are goals that the optimiser tries to achieve). Examples of constraints and objectives are given below in tables 1a and 1b which together form a 'wish-list'.

The cost function 102, together with the model 101, the parameters 103, constraints 105 and reference objective 104, define the problem to be solved.

The cost function 102, constraints 105, reference objective 104, either alone or in any combination, may be referred to as a treatment-planning objective. The treatment-planning objective may be associated to an anatomical structure. In other words, a treatment-planning objective may comprise any of cost function 102, constraint 105, reference objective 104. An example of a treatment-planning objective may be stated as "97% of the volume of a PTV should receive a dose of at least 30 Gy, using a "Target Penalty" cost function". Other examples of treatment-planning objectives are listed in Table 1a.

The optimiser 107 aims to find a set of optimised parameters 111 for which the cost function 102 is minimised. The optimised parameters 111 are an output of the optimiser 107. In an example, when a reference objective 104 is present, the optimiser 107 minimises the difference between the goal value 113 (e.g., the output of the optimiser) and a reference objective 104.

Optionally, the optimiser 107 outputs the cost function value 109. The cost function value 109 is the value of the cost function 102, when evaluated with the optimised parameters 111.

Optionally, the optimiser 107 outputs an achieved goal value 113. The achieved goal value 113 is comparable to the reference objective. An achieved goal value 113 represents a value that has the same units as a given reference objective. The achieved value 113 may be different from the cost function value 109. Unlike the cost function value 109, which may be a number that is an evaluation of the cost function, the achieved value 113 may have a physical meaning. In an example, when the reference objective comprises a dosimetric objective in Gray (Gy), the achieved goal value also relates to a dose (e.g., it has the units of Gy and/or the same physical meaning as the reference objective). In an example, when the reference objective comprises a reference volume and a dose value, the achieved goal value also relates to a reference volume and a dose value (i.e. it has the same physical meaning as the reference objective). As will be described below in relation to Tables 1a and 1b, a reference objective may be DVH-based and may be for a percentage of a volume to receive a predetermined dose. The cost function value 109 may be a number that corresponds to a cost function evaluated with a set of optimised parameters. The achieved goal value 113 would be the achieved percentage of the volume that receives the predetermined dose. In other words, the achieved goal value 113 is comparable to the reference objective.

Fig. 1B shows an example of a two-stage optimisation procedure 150 for IMRT. Although this example applies to IMRT, it is noted that optimisation procedure 100 may be applied to other modes of radiotherapy. For example, the optimisation procedure may also be applied to volumetric modulated arc therapy (VMAT).

In IMRT, one or more radiation beams are directed to a tumour. The intensity of each beam profile is non-uniform. The aim of optimisation procedure 150 is to modify the intensity profile such that a high enough dose is delivered to the tumour, while reducing the dose delivered to healthy organs. The method of optimisation for IMRT 150 comprises two stages. The first stage 151 is fluence map optimisation (FMO) and the second stage 153 is the determination of a configuration of the radiotherapy system. In FMO (stage 151), an optimal fluence map is determined. The optimal fluence map is then used to determine a configuration for the radiotherapy system in stage 2. FMO step 151 will be described next. Step 153 will be described further below.

For fluence optimisation, each beam is divided into a number of beamlets. The contribution of each beamlet, at unit fluence, to voxels is then calculated. Initially, the beamlets may be equally weighted and may contribute equally to the dose distribution. During optimisation, the weight of each beamlet is adjusted such that a cost function is minimised. By multiplying the weight with the contribution of each beamlet at unit fluence and then summing for all beamlets, the full dose distribution may be obtained. The full dose distribution may be compared with any constraints and/or reference objective to determine if the optimised solution is suitable.

The optimisation procedure for FMO may also be expressed as follows. The optimisation procedure for FMO may be performed by the optimisation procedure 100 described herein.

Optimisation comprises minimizing a cost function f(x) by determining suitable values of parameters x based on certain constraints g. For FMO, the parameters x correspond to the weight of the beamlets (x may also be referred to as decision variables, which are the parameters that may be controlled). The output of FMO (Stage 151) comprises parameters x.

The constraints g comprise restrictions. An example of a restriction is a minimum dose at voxels corresponding to a target, or a maximum dose at voxels corresponding to OAR, etc.

In an example, a cost function f(x) is:

$$f(\mathbf{x}) = T1 + T2 + ... + T3$$

where, e.g., $T1 = \Sigma\, \mathbf{x}_n \cdot \mathbf{d}_n$, where, when x corresponds to weight of a beamlet, $\mathbf{d}_n$ represents the dose that each beamlet gives to a voxel at unit intensity. $\mathbf{d}_n$ is a non-optimizable parameter (e.g., it may depend on machine configuration and/or properties of the tissue). $\mathbf{d}_n$ may be obtained from the model 101 described above. For example, from the model 101, it is known whether a voxel n belongs to a target, an OAR, etc... In an example, the dose $\mathbf{d}_n$ may be determined by pencil beam algorithms, convolution based algorithms and/or Monte Carlo (MC) based algorithms. Optionally, dose calculations use pencil beam algorithms or convolution based algorithms (which are fast but have reduced accuracy). Further details of how the dose $\mathbf{d}_n$ may be determined are provided in Monaco® Training Guide, Document ID: LTGMON0530 (Elekta AB).

The output of stage 1 may include a dose distribution. This output may be referred to as a treatment plan.

In this example, the cost function f(x) is the sum of the total dose (T1, T2 ... T3) where each of T1, T2... T3 represent the dose on different structures. Note that alternative formulations of the problem to be solved (e.g. by defining different cost functions or constraints) may be used. In an example, the requirement for the dose at a target to be above a certain amount may be formulated as a constraint. Alternatively, such a requirement could be formulated as a reference objective.

Step 151 provides a set of optimised parameters (e.g., beamlet weights x) that would provide an optimum intensity map (or fluence map). To be ready for delivery by the radiotherapy system, a further step (step 153) is required to translate each optimised beamlet weight into a configuration of the machine that would deliver the optimum fluence.

Determining a configuration (step 153) is described next.

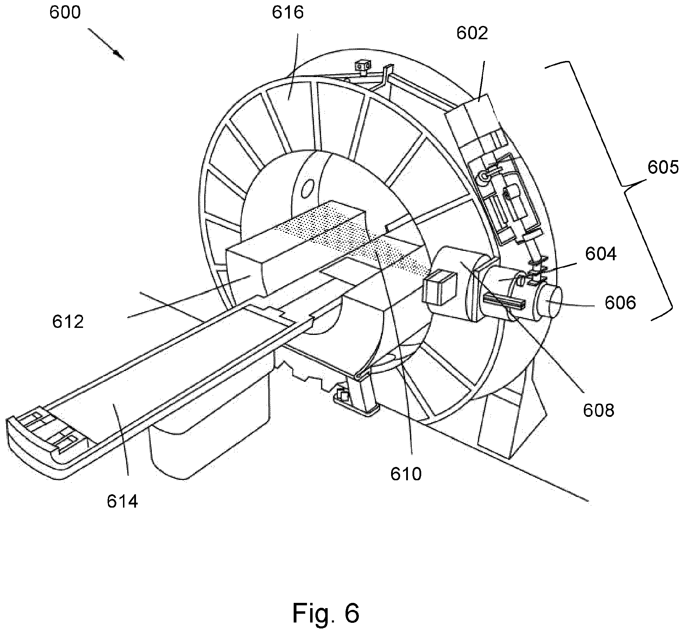

At step 153, the output from step 151 is turned into a configuration of a radiotherapy system. The configuration is useable by a radiotherapy apparatus (examples of which are described herein) for delivering radiation therapy. The configuration may comprise values for each of a set of radiation delivery variables. For example, the configuration of the radiotherapy system comprises a set of (i.e., one or more) aperture configurations. The shapes and weights of the aperture configurations are selected to meet the same goal as in the first stage. Here, shape refers to a shape of an opening. Each shape may be referred to as a segment. Here, weight refers to the weight of each segment. The weight of a segment is related to the dose rate. For a fixed dose rate, the segment weight is proportional to the amount of time the beam is delivered through the segment. For example, a higher segment weight means a longer delivery time through the segment. Thus, the weight of a segment is a function of dose rate and/or the duration for which the beam is delivered through the segment. Aperture configurations may be realised by a beam shaping apparatus, such as part 608 of Fig. 6. When the beam shaping apparatus is an MLC, the shapes are defined by leaf positions.

An aperture configuration may be referred to as a control point or a segment. The control point and/or segment comprise radiation information (e.g. energy, dose) and geometric information such as gantry angle and leaf position.

The shapes and weights of the apertures may be determined by applying algebraic and trigonometric considerations to the arrangement of the aperture in order to implement the optimised beamlet weights of step 151.

Alternatively, step 153 comprises performing a second stage optimisation procedure to determine an optimised aperture configuration that would implement the optimised fluence pattern determined in step 151. The second stage optimisation procedure may be referred to as aperture optimisation, or aperture refinement.

Aperture optimisation may comprise the following:

- Receiving a set of beamlet weights (e.g., from step 151)
- Performing optimisation to determine optimised aperture shapes and, optionally, weights. The optimisation may be performed using an optimisation procedure such as in Fig. 1A, for example. Other optimisation procedures are possible.

When the beam shaping apparatus comprises an MLC, aperture optimisation may comprise:

- Receiving a set of beamlet weights from FMO and/or fluence profiles (e.g., from step 151)
- Converting the received profile into beamlet widths (the opening between a leaf pair). This results in a segment.
- Optimising the weights of the resulting segments.
- Optionally, optimising shapes of the resulting segments (using a procedure referred to as segment shape optimisation).

Note that the optimisation steps in step 153 may comprise a calculation of the dose distribution. The dose distribution may be calculated using pencil beam algorithms, convolution-based algorithms and/or Monte Carlo (MC) based algorithms. Optionally, dose calculations in step 153 use MC based algorithms (which are more accurate but computationally expensive).

Further details of aperture optimisation are provided in Monaco® Training Guide, Document ID: LTGMON0530 (Elekta AB).

Tables 1a and 1b (below), together, show an example list of goals that can be used to guide automated radiotherapy treatment plan generation. Table 1a depicts a set of fixed constraints, whereas table 1b depicts objectives with assigned priorities.

Table 1a

| Structure | Type | Limit |
|---|---|---|
| Patient | Max | 120% of prescribed dose |
| PTV shell | Max (2cm from target) | 50% of prescribed dose |
| Esophagus | Max | 40Gy |
| Spinal Canal | Max | 37Gy |

(continued)

| Structure | Type | Limit |
|---|---|---|
| Trachea | Max | 40Gy |

Table 1b

| Structure | Type | Priority |
|---|---|---|
| PTV | Target Penalty | 1 |
| Patient | Conformality QO | 2 |
| Lung-Target | Mean Dose | 3 |
| Trachea | gEUD | 4 |
| Esophagus | Mean Dose | 5 |
| Heart | Mean Dose | |
| Great Vessels | gEUD | 7 |
| Thoracic Wall | Max | 8 |
| Patient | Conformality QO | 9 |

Together, the constraints of table 1a and objectives of table 1b form an example "wish-list" which can be used to guide an optimisation process to generate a radiotherapy treatment plan. Suitable optimisation algorithms include multi-criterial optimisation algorithms. Examples of suitable multi-criterial optimisation algorithms include the iCycle and mCycle algorithms. iCycle was originally implemented by researchers at Erasmus University Medical Center (Rotterdam, The Netherlands) and re-implemented as mCycle by Elekta Inc. (Stockholm, Sweden; and St. Charles, MO USA). The algorithms are described in detail in the literature, for example in:

- Breedveld S, Storchi PR, Voet PW, Heijmen BJ. iCycle: Integrated, multicriterial beam angle, and profile optimization for generation of coplanar and noncoplanar IMRT plans. Med Phys. 2012 Feb;39(2):951-3. doi: 10.1118/1.3789. PMID: 22220804
- Bijman R, Sharfo AW, Rossi L, Breedveld S, Heijmen B. Pre-clinical validation of a novel system for fully-automated treatment planning. Radiother Oncol. 2021 May;158:253-21. doi: 10.101/j.radonc.2021.03.003. Epub 2021 Mar 10. PMID: 33711413.

The optimiser takes a model such as a segmented image of the patient as an input. The image depicts a target volume which is to receive a prescribed dose of radiation during the treatment. The target volume may be a planning target volume, PTV, which encompasses the tumour and an extra volume around the tumour to account for potential uncertainties in the treatment, e.g. patient position. The segmented image is segmented, or 'contoured', to show the tumour and/or the PTV, and any surrounding OARs. The optimiser also takes the wish list as an input, which comprises both the 'hard' or 'fixed' planning constraints (e.g. such as those depicted in table 1a) and the objectives (e.g. such as those depicted in table 1b), where each objective has an ascribed priority and is ranked in order of importance according to the ascribed priorities to provide a guide for the optimisation process.

Based on this input, a fluence map optimization (FMO) may be performed using a multi-criterial optimiser in a known way. Each of the first set of objectives has an ascribed priority and the first set of objectives is ranked in order of importance according to the ascribed priorities to provide a guide for the optimiser.

First, the 'fixed' constraints are used in a first iteration in order to generate an intermediate plan. Then, the wish list objectives are introduced in subsequent iterations of the optimiser. In this way, intermediate plans are generated by consecutive optimisation of objectives in order of their associated priorities. The final plan is established once the final objective has been introduced and optimised, resulting in a final treatment plan that takes into account all the wish-list constraints and added objectives. In this way, the radiotherapy treatment plan is generated, at least in part, by sequentially optimising for the objectives in turn according to their ascribed priorities. The FMO optimiser outputs a 3D dose distribution, which can be used as an input into a second stage of optimisation in which the radiation delivery variables are generated in a known way.

As used herein, "radiotherapy treatment plan" is used primarily to refer to the optimised dose distribution, as represented for example by the 3D dose distribution or the series of 2D dose distributions, and/or by the related 3D fluence map or series of 2D fluence maps.

With reference to table 1a, it can be seen that each constraint comprises an associated structure (e.g. an anatomical structure), a 'type', and a limit. The structure defines the anatomical structure to which the constraint applies, for example a target volume that includes the tumour or a nearby OAR such as the esophagus, spinal canal, and trachea. The 'type' of constraint may be, for example, a maximum dose threshold (i.e. the maximum dose that can be applied to the structure during treatment). The constraints may be relative to a prescribed dose, or may be an absolute value e.g. in Gy.

In the first row of table 1a, a constraint is defined that for the patient, i.e. that for the entire patient volume, the maximum dose must be under 120% of the prescription dose. The second row indicates that a dose applied to a PTV shell, or PTV ring, must be under 50% of the prescription dose. This ensures a rapid fall-off of dose outside the PTV to better protect surrounding healthy tissues or organs-at-risk (OARs)

With reference to table 1b, it can be seen that each objective also comprises a structure and a type. The objectives also comprise a limit or 'goal' in a manner similar to the constraints, though these are omitted in this example table for brevity. 'Types' of constraint or objective include maximum dose, minimum and mean dose to be applied during treatment. Other types of constraint / objective comprise a target penalty, which is a type of constraint / objective that seeks to ensure that the target volume receives the prescribed dose. It penalizes underdosing or overdosing within the target volume (in the case of table 1b the PTV). Conformality Quality Objective (QO) is a measure used to evaluate how well the radiation dose conforms to the shape of the target volume. Equivalent uniform dose (EUD) and generalised EUD (gEUD) are metrics that characterize the effects of non-uniform dose distributions.

The objectives additionally comprise ascribed priorities, and are ranked in the table according to the priority values. The priority reflects the relative importance of the objective. The higher a priority is, the higher the probability that the corresponding objective will be met during the optimisation. MCO algorithms may be used to generate one or more Pareto-optimal solutions, where no single objective can be improved without worsening a constraint or another objective. At a high-level, during each iteration, an appropriate stopping criterion may cause the optimiser to stop when it hits a point when it needs to violate one or more higher priority objectives in order to seek further improvement. For example, inspection of tables 1a and 1b will reveal that, during a multi-criterial optimisation process based on this wish-list, delivering a sufficient dose to the tumor is given higher priority than ensuring the thoracic wall doesn't exceed a maximum dose. The wish list, defined by a set of constraints and a set of ranked objectives, helps guide the optimisation process towards achieving optimal outcomes based on the defined constraints, objectives and their associated priorities.

Radiotherapy can damage healthy tissues and organs, leading to various side effects. The present application relates to a method in which symptom data, i.e. data which is indicative of the existence or severity of a symptom experienced by a radiotherapy patient, is incorporated into the treatment planning process. For example, during delivery of a course of radiation according to an initial radiotherapy treatment plan, the patient may report the existence of a new symptom, or the worsening of an existing symptom. If this development is not expected for the patient at the current stage of their plan, it may be inferred from the symptom data that a particular anatomical structure associated with the symptom has received too much radiation dose during treatment, or else that a reduction in the amount of radiation the anatomical structure is set to receive in future treatment sessions would be beneficial. The emergence of particular symptoms / side effects is not straightforward to predict at the initial planning stage, not least because different patients experience different levels of radiosensitivity.

Table 2

| Symptom(s) | Associated anatomical structure(s) |
|---|---|
| Redness, itching, dryness, peeling, blistering, or darkening of the skin in the treated area. | Skin |
| Hair Loss in the area where radiation is directed | Skin (e.g. scalp) |
| Nausea and/or vomiting, Loss of appetite | Gastrointestinal system (stomach, intestines) |
| Diarrhoea, blood in stool | Gastrointestinal system (intestines), rectum |
| Dry mouth, sore throat, difficulty swallowing, changes in taste. | Oral cavity and throat (pharynx) |
| Persistent cough, difficulty breathing, shortness of breath, wheezing, and stridor. | Lungs, trachea |
| Frequent urination, burning sensation during urination, blood in urine, incontinence | Bladder |
| Chest pain, heart palpitations. | Heart |

Table 2 provides a non-exhaustive list of example radiotherapy symptoms (e.g. side effects) and associated anatomical structures. Delivery of radiation to these structures can cause the associated symptoms.

In some examples, the symptom data is self-reported patient data. For example, the symptom data may be data collected from patient responses to questionaries regarding the symptoms / side effects they are experiencing throughout their treatment.

The patient-reported data may be collected via a questionnaire. The questionnaire provides a series of questions related to the patient's treatment, with the aim of determining whether the patient is experiencing any new symptoms, or whether the severity of any of the patient's existing symptoms has worsened. The series of questions may be standardised, or customised to the patient and their treatment. The questions may ask for "yes/no" answers regarding the existence of a particular symptom, may ask the patient to rank the severity of a symptom on a scale (e.g. from 1-10), or the patient-reported symptom data may be provided as unstructured data collected in one or more text inputs of the questionnaire.

Answers to the questionnaire may be collected via a user interface of a a mobile device, e.g. via a software application and/or a web-based interface. Such interfaces may be used to collect data in a conversational (e.g., text chatbot) or questionnaire interface, which provides a series of questions. For instance, a questionnaire interface may offer questions that are displayed or change based on previous responses, as structured data is collected from a patient in the questionnaire answers. Also for instance, a questionnaire interface may offer an open-ended text field which is used to collect freeform, unstructured text answers or responses from a patient. Other interface types, including the collection of data from wearable devices, may also be used.

Examples of suitable questions which may be included in a questionnaire in order to collect the symptom data include:

- In the past 7 days, have you had pain in the treatment area?
- What has been the severity of that pain, on a scale from 1-10?
- Have you experienced radiation-induced skin changes?
- Have you experienced shortness of breath?
- Have you experienced difficulties in swallowing?
- Have you experienced any of itching, fatigue, joint pain, dizziness, chest pain, or stomach pain?
- How severe are these symptoms, in your opinion, on a scale from 1-10?
- Which of these symptoms have worsened over the last 7 days?
- Which have improved over the last 7 days?

The questions may include selectable, multi-choice questions, and there may be question input controls (e.g., a slider). The questionnaire may adapt the questions according to the patient's responses, e.g. if the patient is not experiencing chest pain, they will not be asked further questions about chest pain.

The symptom data may comprise other information such as patient data measurements from the mobile device itself or accompanying wearable devices (e.g., number of steps determined from a smart watch or phone).

In some examples, additional processing may be used to convert raw symptom questionnaire answer data into graded electronic patient reported outcome (ePRO) data, such as with the use of an algorithm that grades individual symptoms indicated in the questionnaire raw data. Such grading may be based on some standardised metric or standard (such as international standards relevant to the condition or treatment). By comparison with standards or patient data gathered from patients undergoing similar treatment, it can be determined whether a symptom is expected, or not expected, for the patient.

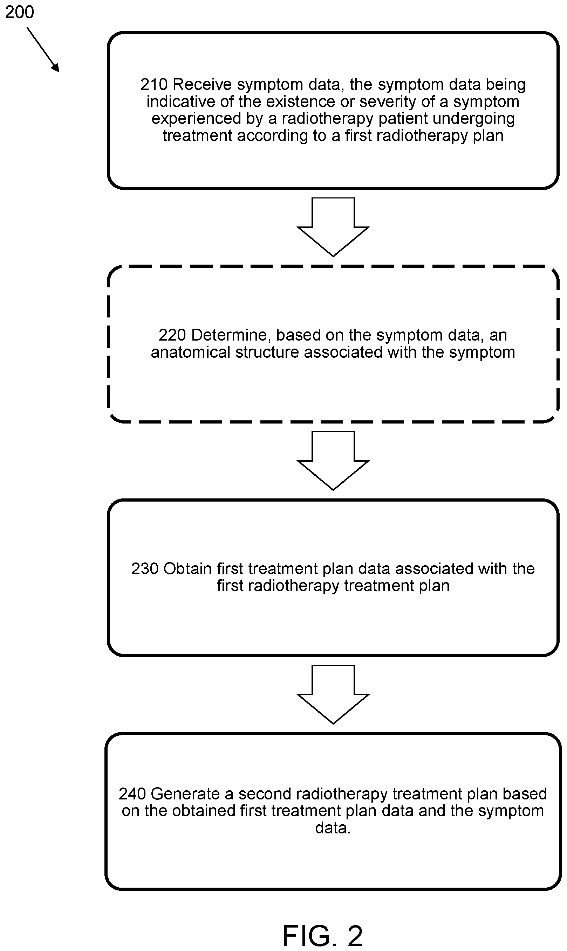

Figure 2 depicts a method 200 according to the present application. The method 200 is a method suitable for adaptive radiotherapy. For example, the method 200 is suitable for adapting the treatment plan of a patient based on self-reported symptom data in order to reduce the radiation dose applied to an anatomical structure, for example an organ, which is associated with the symptoms described by the symptom data.

At block 210, symptom data is received. The symptom data is indicative of the existence or severity of a symptom experienced by a radiotherapy patient undergoing treatment according to a first radiotherapy plan.

The symptom data may comprise, or be based on, symptom data captured by a clinician, for example in regular meetings with the patient. The symptom data may additionally or alternatively comprise, or be based on, self-reported symptom data which has been reported by the patient. For example the patient may report, via a questionnaire or any of the other reporting mechanisms described herein, their experience with side effects and symptoms associated with the delivery of radiation according to the first treatment plan. In an example, the patient may report a severity of '9' on a scale from 1-10 associated with symptoms of 'difficulty breathing' and 'shortness of breath'.

This raw questionnaire data may be the "symptom" data and the existence of symptoms of that level of severity may trigger adaption of the plan.

In some examples, the raw questionnaire data is compared with standards or thresholds, for example accepted international standards, which define expected symptoms and their expected severity rages at various stages of treatment. This comparison enables a determination of whether a particular symptom, and/or its reported severity, is expected at the patient's stage of treatment.

In some examples, while not shown in the flowchart, the method 200 further comprises determining whether the existence or severity of the symptom is expected or unexpected based at least in part on a stage of the first radiotherapy treatment plan. For example, the method may comprise comparing the patient's self-reported symptoms and/or their severity to expected symptom data for the relevant stage of treatment. The 'stage of treatment' might be described by the number of fractions of treatment the patient has received, the cumulative dose received to date by the tumour (e.g. the PTV) or a particular OAR. If the symptom is expected, the method 200 may halt or discontinue, as no adaption of the first treatment plan is required. If however the existence or severity of the symptom is unexpected, then the method proceeds and, ultimately, a second radiotherapy treatment plan is generated.

Optionally, at block 220, an anatomical structure associated with the symptom is determined, e.g. identified, based on the symptom data. In an example, a look-up table, LUT, may be stored in memory which maps symptoms and/or their severity to associated anatomical structures. This LUT may take the same or a similar form to table 2 above. The method 200 may comprise determining the anatomical structure based on the look-up table, e.g. by consulting the LUT.

LUTs are just one example way in which the anatomical structure can be identified based on the symptom described in the symptom data. Ultimately, the aim is to identify the anatomical structure, such as an organ, which is responsible for the symptom. As will be seen, an aim of the method 200 in certain implementations is to identify an anatomical structure associated with the patient's symptoms, and generate a new treatment plan which provides a reduced dose to the identified anatomical structure.

For example, alternatively, an anatomical structure is identified using a pre-trained classifier. The pre-trained classifier is configured to map symptom data to an associated anatomical structure. The pre-trained classifier may be implemented using a machine learning algorithm. Examples of suitable algorithms include: naive Bayes classifiers, support vector machines, or deep learning algorithms. The pre-trained classifier may be trained as follows. The classifier may be trained on a labelled data set comprising text strings of patient reported symptoms and an associated organ. The labelled data set may include between 100 to 10000 data pairs, for example.

At block 230, first radiotherapy treatment plan data is obtained. This data is associated with the first radiotherapy plan.

The treatment data may comprise a model such as the model 101 described above with relation to figure 1. In an example, the first radiotherapy treatment plan data comprises an image such as a radiotherapy planning image of the patient. The planning image may be a computed tomography (CT), cone-beam computed tomography (CBCT), or MR image for example. The planning image depicts a target region, such as a PTV or other planning volume encompassing part or all of a tumour, and the anatomical structure identified at block 220. This planning image may have been contoured (segmented) such that the target region, the determined anatomical structure, and any other anatomical structures of interest such as OARs are labelled in the image. This type of image is a common input into radiotherapy treatment planning procedures and algorithms, as the skilled person will know.

The obtained image may be retrieved from memory, and may be an original reference image on which the first radiotherapy treatment plan was based. Alternatively, the obtained image may be a 'daily' image, i.e. an image of the patient acquired on one of their treatment days. As the skilled person knows, a daily image in radiotherapy refers to the image acquired each day before delivering radiation treatment. The daily image is used to assist with patient positioning and, in some workflows, as an input to adaptive radiotherapy techniques. An advantage of using the latest available daily image is that the new / adapted plan is generated based on the latest available anatomical data, leading to improved accuracy and improved efficacy.

In another example, the first treatment plan data may additionally (or alternatively) comprise a first set of objectives which were used to guide a first optimisation process during generation of the first treatment plan. In this example, the first treatment plan was generated via a multi-criterial optimisation (MCO) algorithm based on a first set of objectives. The first set of objectives may take the form of a wish list of objectives such as the wish list depicted in table 1b and described above. At least one of the objectives of the first set of objectives relates to the anatomical structure identified in block 220. For example, the at least one objective may specify that the determined anatomical structure should receive a mean dose of less than 5Gy, or may specify that the structure should receive an EUD of less than 20Gy. At block 230, the method 200 may additionally comprise determining (e.g. identifying) the at least one objective which is associated with the anatomical structure, e.g. by determining which of the first set of objectives defines a limit or requirement that relates to the anatomical structure.

As described above, the at least one objective also comprises a priority which indicates its relative importance compared to the other objectives in the first set of objectives. Each of the first set of objectives has a priority ascribed to it, and the first set of objectives is ranked in order of importance according to the ascribed priorities. In this way, the first set of objectives provide a guide for a first optimisation process which was used to generate the first treatment plan. The first radiotherapy treatment plan has been generated, at least in part, by sequentially optimising for the first set of objectives in turn according to their ascribed priorities. Optionally, this process also comprises first optimising for a set of fixed constraints, such as those depicted in table 1a and described above.

At block 240, a second radiotherapy treatment plan is generated based on the obtained first radiotherapy treatment planning data and the symptom data. The second radiotherapy treatment plan may be an 'adapted' radiotherapy treatment plan.

The second radiotherapy treatment plan defines a reduced dose to the anatomical structure associated with the symptom compared with a first planned dose defined by the first treatment plan. This is achieved whilst ensuring the dose still meets the defined dose constraints to other organs and ensuring satisfactory target coverage.

The second radiotherapy treatment plan generated at block 240 defines the delivery of therapeutic radiation for at least a next fraction of the patient's treatment, e.g. by defining the dose distribution to be delivered to the patient and/or by defining the radiation delivery variables (i.e. the device configuration data) which should be employed by a radiotherapy device in order to produce the desired dose distribution. Preferably, the second radiotherapy treatment plan defines the delivery of therapeutic radiation not only for a next fraction of the patient's treatment, but also for the patient's remaining treatment.

As described above with respect to block 230, the first treatment plan data may comprise the wish list that was used to generate the first radiotherapy treatment plan. In this example, generating the second radiotherapy treatment plan at block 240 may comprise adapting the first set of objectives to form an adapted set of objectives. The first set of objectives may be adapted in several ways, with the ultimate aim of reducing the dose applied to the anatomical structure in a second treatment plan generated based on the adapted set of objectives. For example, where there is at least one objective on the initial wish list that relates to the anatomical structure, the at least one objective can be adapted by adjusting any one or more of its ascribed priority, its type, or a goal or limit associated with the objective.

In an example, adapting the at least one objective comprises increasing its priority. This has the effect of making the objective more 'important' in the optimisation process. As a result, the objective is therefore more likely to be met, or at least the objective will move closer toward being met in comparison with the first radiotherapy treatment plan. In an example, the at least objective may have its priority increased by a predetermined number, e.g. by 1 or by 2. In another example, the at least objective associated with the affected organ is reprioritized to a predetermined position in the priority ranking, for example to be just below the objectives which are associated with the target and/or with the PTV.

The manner in which the at least one objective is adapted may be determined by a function, such as a trained model or an algorithm. The function takes some or all of the obtained first treatment plan data and the symptom data as an input, and outputs data comprising an adapted set of objectives which can form the basis of an optimisation process to generate the second radiotherapy treatment plan. For example, this function may be designed to take into account the severity of the side effect while generating the adapted set of objectives. For example, more serious (severe) side effects may result in the associated objective(s) being moved up the priority ranking to a priority just below the target or PTV, whilst moderate side effects result in an increase of 1-2 places in the priority rankings. Similarly, the function could take certain aspects of the patient's self-reported symptom data as an input to determine that the 'type' of the objective should be changed, for example to change a dose uniformity objective such as an EUD type objective to a "maximum dose threshold" type objective, or vice versa.

The new, 'adapted', second set of objectives comprises a new objective and/or an adapted at least one objective, and the remaining (other) objectives of the first set of objectives. Each objective of the resulting adapted set of objectives has an ascribed priority and is also ranked in order of importance according to the ascribed priorities to provide a guide for a second optimisation process. Optionally, as described above, the at least one objective associated with the anatomical structure determined at block 220 has an increased priority in the second set of objectives in comparison with its priority in the first set of objectives.

In some circumstances, the first set of objectives may not comprise an objective which relates to the determined anatomical structure, i.e. the anatomical structure determined to be responsible for the symptom indicated by the symptom data. If it is determined that the first set of objectives does not comprise an objective which relates to the determined anatomical structure, then the first set of objectives may be adapted by adding at least one new objective. The at least one new objective does relate to the anatomical structure. In this way, the adapted set of objectives comprises one or more new objectives which aim to reduce the dose which will be applied to the anatomical structure. In this case, an objective could be automatically added based on a library or database of potential objectives which are typically applied to the anatomical structure. A suitable objective may comprise, for example, an objective that aims to reduce the mean dose to the structure.

The method 200 then comprises performing a (second) optimisation process based on the adapted, 'second' set of objectives. Optionally, the second optimisation process may additionally be based on the same, unchanged set of fixed constraints as were used to generate the first radiotherapy treatment plan.

Optionally, the adapted set of objectives may be generated by both adjusting an existing objective which relates to the anatomical structure, and additionally adding one or more new objectives which relate to the structure. For example, an objective of a particular type may always be added to the wish list such that, if the existing objective is not of the particular type, then a new objective of the particular type is added in addition to the existing objective having its priority increased.

Optionally, at block 240, multiple plans may be generated, such that the second radiotherapy plan is a plurality of adapted radiotherapy treatment plans. Each plan of the plurality of adapted radiotherapy treatment plans may be generated based on the same wish list comprising the adapted set of objectives, in which case each plan defines a different trade-off between the various objectives of the adapted set of objectives. Each plan of the plurality of adapted radiotherapy treatment plans may therefore comprise a different dose distribution, which differs from the dose distribution of the first radiotherapy treatment plan. Each dose distribution defines a reduced dose to the anatomical structure associated with the symptom, but this reduction in dose is achieved in different ways. For example, the dose distribution defined by each plan may be less conformal than the distribution of the first treatment plan. In each of the adapted radiotherapy plans, the change in conformality may take different forms, e.g. some distributions may give more dose to unspecified tissues such as fats and muscles but via different spatial distributions. In this way, a series of pareto-optimal plans are presented to the reviewer, so they can evaluate which one is most suitable for the patient. Using existing automated evaluation techniques based on each plans dosimetric criteria, a recommended or 'suggested' plan may also be presented to the clinician.

The generated plan may take the form of dose distribution data describing the optimised spatial distribution of dose in the patient's anatomy. The dose distribution defined in the second radiotherapy treatment plan is such that less dose is delivered to the anatomical structure associated with the unexpected symptom. Optionally, the second radiotherapy treatment plan may additionally comprise values for one or more radiation delivery variables which, when implemented by a radiotherapy device, will result in a dose distribution according to the intended dose distribution. These variables are described elsewhere herein and may include one or more of: a weight of a particular beam, the beam energy level and dose rate, the beam shape (e.g. defined by positions of multi-leaf collimator leaves for a particular beam); gantry angles at which radiation is to be delivered, beam on and off times, and the like. In other words, the second treatment plan may be a plan which takes the form of a dose distribution after fluence map optimisation 251, or a plan as defined after a corresponding device configuration 253 has been determined.

After block 240 has completed and at least one second radiotherapy treatment plan has been generated, several optional next steps may occur. The plan(s) generated at block 240 may be sent to a clinician to allow the clinician to confirm that the new plan is acceptable for delivery to the patient. In an example in which multiple new plans are generated, each plan may be sent to the clinician to enable them to select which plan should be delivered to the patient. The clinician may be sent second treatment plan data, for example, which comprises one or more dose distributions associated with one or more adapted treatment plans, the differences between each plan and the original ('first') treatment plan, and the symptom data that triggered the generation of the new plans. A comparison of the reference (first) plan to each of alternative plans may also be presented. The clinician is able to review the second treatment plan data and decide which, if any, of the adapted plans should be delivered to the patient.

Once the second radiotherapy treatment plan has been generated, and optionally once the clinician has confirmed it should be delivered to the patient, then the method may additionally comprise delivering radiation to the patient, via a radiotherapy device, according to the second radiotherapy treatment plan. As discussed above, the second radiotherapy treatment plan may comprise a desired, e.g. optimised, dose distribution, which is suitable for inputting into a second optimisation process as described above with respect to figure 2 in order to generate radiation delivery variables based on the desired dose distribution. The method may comprise delivering the treatment, using the radiation delivery variables, to the patient.

The method 200 may vary in a number of ways to the specific examples described above. For example, as described above in relation to block 230, the method 200 may comprise determining the objective in the first set of objectives which is associated with the relevant anatomical structure. However, in some instances, the first treatment plan may have been generated based on a set of objectives which contains more than one objective which relates to the anatomical structure. In this scenario, each of the relevant objectives is identified within the first set of objectives. The objectives are adapted based on a pre-determined framework. In an example, the objectives are each adapted, both in isolation and in combination, to generate a plurality of second (adapted) sets of objectives, which are each used to generate new plans. For example, in the case of two relevant objectives, one or more first treatment plans are generated based on a set of objectives in which both the objectives are prioritized; one or more second treatment plans are generated based on a set of objectives in which a first one of the two objectives is prioritized; and one or more third treatment plans are generated based on a set of objectives in which the second one of the two objectives is prioritized. Each of these treatment plans can be sent to the clinician for comparison. This process is quick from a computational perspective. The clinician can then select which of the several plans to proceed with.

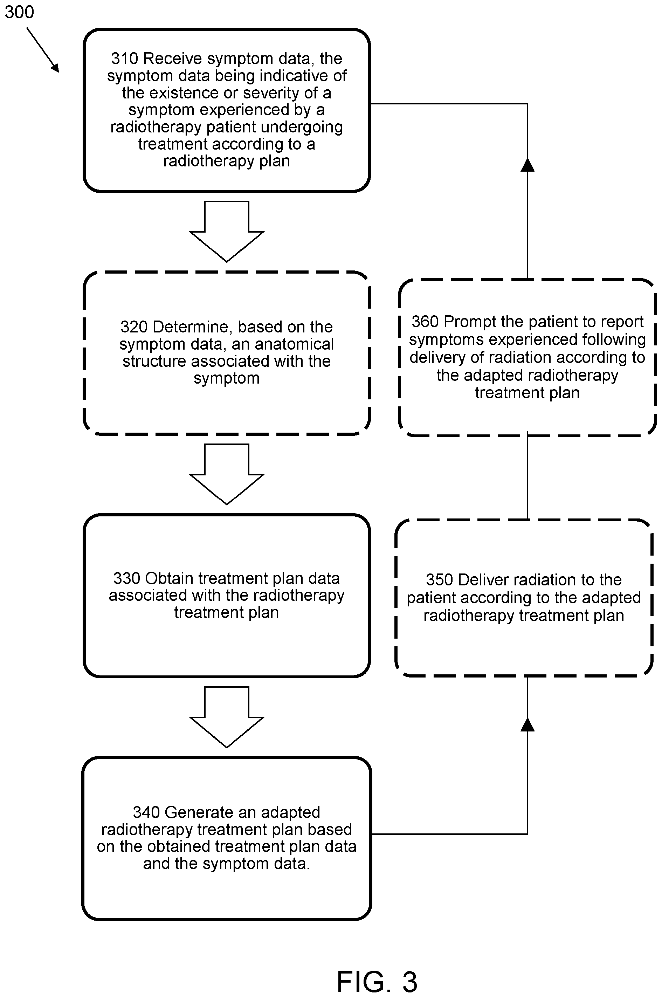

It should also be noted that the method 200 may be an ongoing process during a patient's treatment. Figure 3 depicts a method 300 according to the present disclosure. Method 300 can be described as an adapted version of method 200 which allows for continuous updates of a radiotherapy treatment plan based on regularly received symptom data. Like reference numerals are used to refer to like stages of the method, and the methodologies are the same or similar unless otherwise specified.

At block 310, symptom data is received and at block 320, an anatomical structure may be determined which is associated with a symptom described by or otherwise associated with the received symptom data. At block 330, treatment plan data is obtained which is associated with the treatment plan currently being delivered, for example the plan which defined how radiation was delivered to the patient during their most recent treatment session. At block 340, an adapted radiotherapy treatment plan is generated in the same or a similar way as that described above with respect to block 240.

Optionally, at block 350, radiation is delivered to the patient according to the adapted radiotherapy treatment plan. Block 350 may comprise delivering radiation to the patient in one or more fractions in accordance with the plan.

Optionally, at block 360, the patient may be prompted to report any symptoms experienced following the delivery of radiation according to the adapted radiotherapy treatment plan. After generation and delivery of the second (adapted) radiotherapy plan, the patient's initially reported symptoms and their severity may not improve, and/or they may develop new symptoms or experience changes in severity in a different symptom. At block 360, they are prompted to report these symptoms to produce new symptom data. The new symptom data may be indicative of the existence or severity of a new symptom experienced by the patient undergoing treatment according to the adapted radiotherapy plan, or may be indicative of a change in either the existence or severity of the original symptom.

Block 360 may comprise issuing a notification to the patient's mobile device, for example, requesting that they fill out a questionnaire or otherwise report their symptoms via a mobile software app or web-based application in the manner described elsewhere herein.

To provide an example of the application of method 200 and closely related method 300, consider a patient undergoing radiation therapy for non-small cell lung cancer located high in the chest. The patient is undergoing radiotherapy according to a first radiotherapy treatment plan. This plan has been generated in a known way using an MCO approach based on a first wish list comprising a first set of ranked objectives. The patient has had 3 sessions of radiotherapy treatment to date, and is scheduled for 3 more.

The patient experiences several symptoms during the course of their treatment, including shortness of breath, but after the most recent fraction of their treatment they additionally begin experiencing wheezing and stridor.

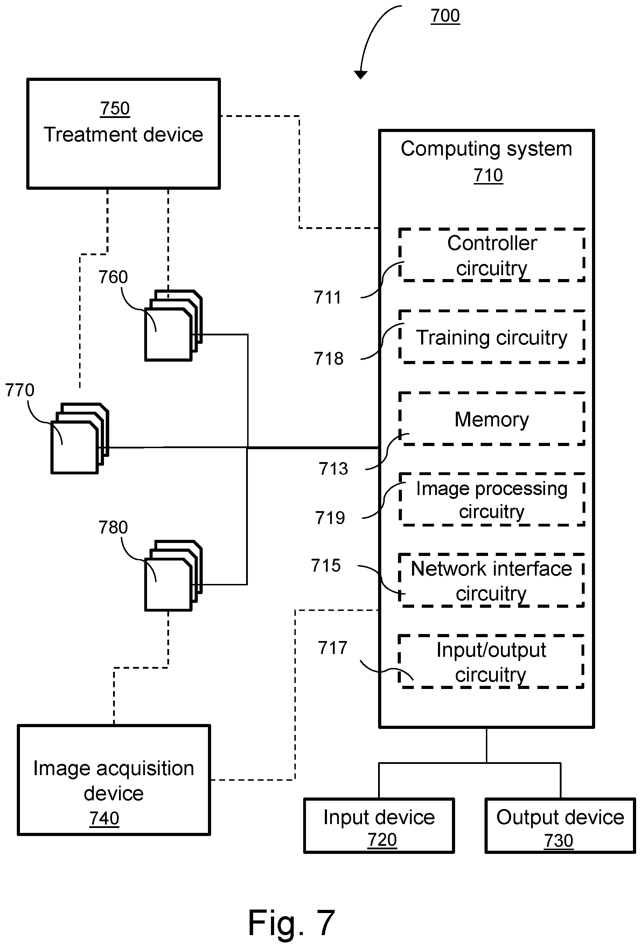

The patient reports their symptoms via a mobile software app on their phone. For example, they may indicate that the shortness of breath is still present but has not worsened. The patient also reports the existence of two new symptoms, wheezing and stridor, and that in their opinion each of these symptoms has a severity of 7 out of 10. The raw questionnaire data is compared to pre-set thresholds and it is determined that shortness of breath is an 'expected' symptom, based on the stage of the patient's treatment (3/6 fractions) and based on the type of treatment the patient is receiving. However, both the wheezing and stridor and their reported severity are determined to be 'unexpected' by comparison with expected symptoms and expected severity levels. This symptom data, relating to the unexpected symptoms, is received by a treatment planning system. The treatment planning system may take the form of a computing system such as the computing system 710 described below with respect to figure 7.

Upon receipt of the symptom data, the treatment planning system determines, based on the symptom data, an anatomical structure associated with the reported symptoms. It is determined that the trachea may be responsible for these symptoms, and in particular that the dose received by the trachea to date during treatment may have damaged the trachea leading to the symptoms of wheezing and stridor.

The treatment planning system obtains first treatment plan data associated with the patient's treatment plan from memory e.g. memory 713. The treatment planning system retrieves both the contoured reference image that formed the basis of the patient's treatment plan, and also retrieves the wish list which was used to generate the dose distribution defined in the plan.

The treatment planning system identifies two objectives in the original wish list that define a goal relating to the trachea. The first of these objectives is a maximum dose objective which stipulates that the maximum dose to the trachea should not exceed 30 Gy. The second of these objectives is a mean dose objective that specifies that the mean dose to the trachea should be kept below 20 Gy.

The treatment planning system increases the priority of each of these objectives by 1 factor, thereby generating a new, adapted wish list based on the original wish list. In this example, the adapted wish list has the same objectives as before, but they are ranked in a different priority order.

Once an adapted wish list has been created, the treatment planning system generates multiple new fluence maps by performing an MCO optimisation process multiple times. Each map is generated, at least in part, by sequentially optimising for the objectives in the adapted wish list, in turn, according to their ascribed and newly updated priorities. Each of the new fluence maps meets all the objectives in the adapted wish list, while comprising both a lower maximum and mean dose to the trachea. The fluence maps are sent to a clinician to enable them to select which dose distribution should be delivered to the patient. The selected fluence map undergoes a second stage of optimisation in order to generate radiotherapy delivery variables which are used to define the delivery of radiation to the patient in their next fraction of treatment.

Another example of the presently disclosed teachings may be applied to a scenario in which a radiotherapy patient experiences blood in their stool. The method may comprise not only identifying one or more existing objectives within the current wish list and increasing their priority, but also determine whether a particular kind of objective associated with the anatomical region is present in the wish list and, if not, adding a new objective. For example, the method may comprise, at block 240, determining whether the wish list associated with the first treatment plan comprises any objectives which are i) associated with the anatomical region, and ii) are of a particular type or goal. If the determination under either i) or ii) is negative, a new objective can be added which is associated with the anatomical region and which comprises the particular type or goal.

For example, a patient is experiencing blood in their stools while undergoing treatment according to a first treatment plan. The rectum is identified as the anatomical structure associated with this symptom. The wish list used to generate the patient's treatment plan contains an objective associated with the rectum, but there is no maximum dose objective associated with the structure. In order to generate an updated (second) radiotherapy treatment plan, the priority of the existing objective associated with the rectum is increased. In addition, a new maximum dose objective associated with the rectum is added to the wish list. In this way, the dose which will be delivered to the rectum during implementation of the adapted plan will be decreased significantly compared with the dose that would be delivered to the rectum according to the patient's original plan.

Figures 4a-c depict 2d dose distributions. The dose distributions have been generated by three different optimisation processes, each based on a different wish list. The dose distributions are segmented to depict a tumour 410, the rectum 420, and the bladder 430. Also depicted is a contour depicting the patient's right femur 440. Each of figures 4a-c also depicts cumulative dose via a greyscale in units of Gy.

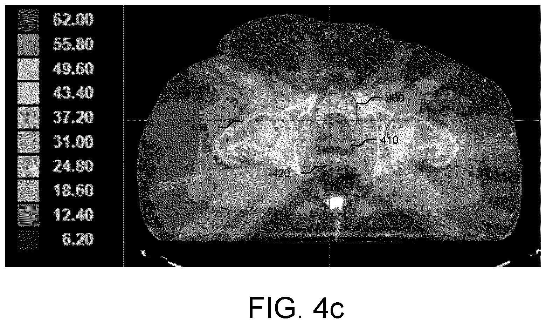

The example dose distributions are to be applied to a patient with prostate cancer. Figure 4a depicts an optimised dose distribution generated based on a first wish list. This dose distribution forms part of a first treatment plan; a reference plan. This reference plan meets all of the constraints of the first wish list and the depicted dose distribution has been produced by optimising for the list of objectives contained within this wish list in priority order. Figures 4b and 4c depict two alternative, adapted plans for the same patient created based on patient-reported symptoms. The alternative plans maintain the accepted dose within the target, i.e. to the tumour 410, but reduce ALARA doses to the bladder, rectum, and to the combined bladder + rectum area. This adaptive planning task is accomplished in an automated manner through the auto-planning wish list objective prioritization mechanism described herein.

Initially, the patient is undergoing treatment according to the reference treatment plan associated with the dose distribution depicted in figure 4a. After 5 of 20 planned fractions, the patient reports through a patient monitoring software application that they are experiencing extreme diarrhoea. It is possible that radiation dose delivered to the patient's rectum is responsible for this symptom.

Whilst this is a potential side effect of their treatment, it is not expected this early in the treatment course. According to the methods described herein, a new plan is generated and presented to the physician. A notification is sent to the physician through a physician interface of the software application. The physician chooses to use this plan for the next fraction of the patient's treatment.

A dose distribution according to the new, adapted plan is depicted in figure 4b. As can be appreciated, the new plan defines a reduced dose to the rectum 420 compared to the plan depicted in figure 4a. This plan has been generated by increasing the priority of an objective relating to the rectum, and thereby de-prioritising objectives relating to intermediate dose conformality. The dose to the rectum 420 represented by the distribution in figure 4b is far below acceptable limits in order to help alleviate the patient's reported symptoms, at the expense of intermediate dose conformality which is extremely unlikely to have a significant impact on the efficacy of the patient's treatment.

After delivery of one or more fractions of the (first) adapted plan represented by the dose distribution in figure 4b, i.e. the adapted plan generated in order to spare dose to the rectum, the patient later reports that they are becoming incontinent. This is likely associated with dose being delivered to the bladder during treatment. Based on this reported symptom, another (second) adapted plan is generated which not only seeks to reduce the dose delivered to the rectum to address the patient's reported symptoms of diarrhoea, but also seeks to reduce the dose applied to the bladder in order to address the patient's newly reported symptoms.

A dose distribution according to the second adapted plan is depicted in figure 4c. In this plan, not only is there reduced dose applied to the rectum 420, but also to the bladder 430. Again, this comes at the expense of the dose conformity. However, the reduced dose conformity is clinically acceptable, and it is expected that downsides associated with a resulting increased dose in, for example, unspecified tissues such as muscle and bone will be far outweighed by the benefits of an alleviation in the patient's reported symptoms.

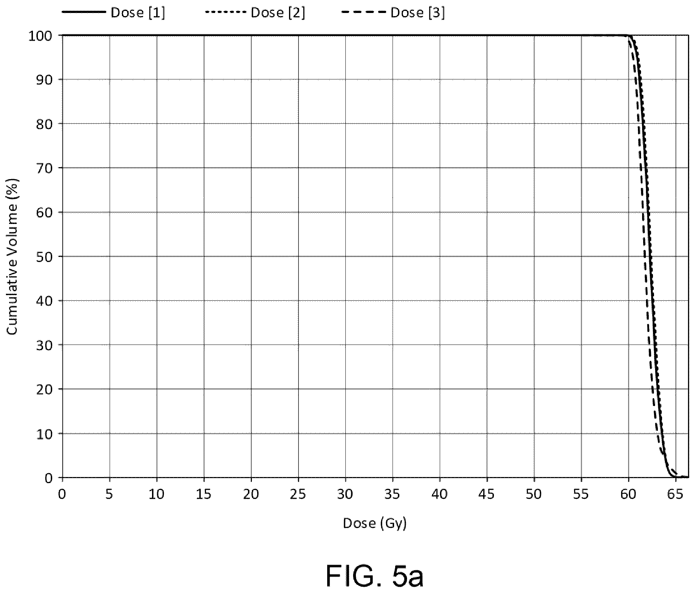

Figures 5a to 5c depict the doses applied to each of the tumour, rectum, and bladder according to the dose distributions / plans depicted in figures 4a-c. Each of figure 5a-c is a graph plotting cumulative volume by percentage on the y axis and dose in Gy along the X axis. This type of graph may be referred to as a dose-volume histogram.

In each graph, the doses relating to the reference plan depicted in figure 4a are depicted in a thinly dotted line (dose [2] in the graph's key), the doses relating to the first adapted plan depicted in figure 4b are depicted via an unbroken line (dose [1] in the graph's key), and the doses relating to the second adapted plan depicted in figure 4c are depicted in a dashed line (dose [3] in the graph's key).

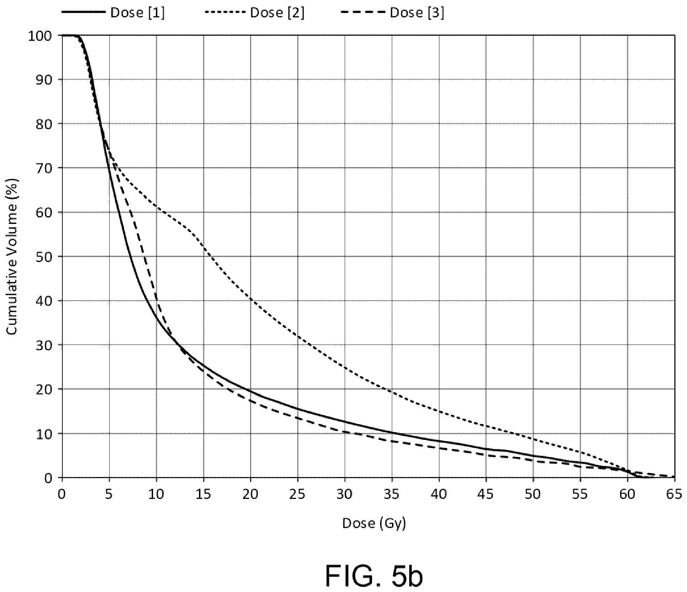

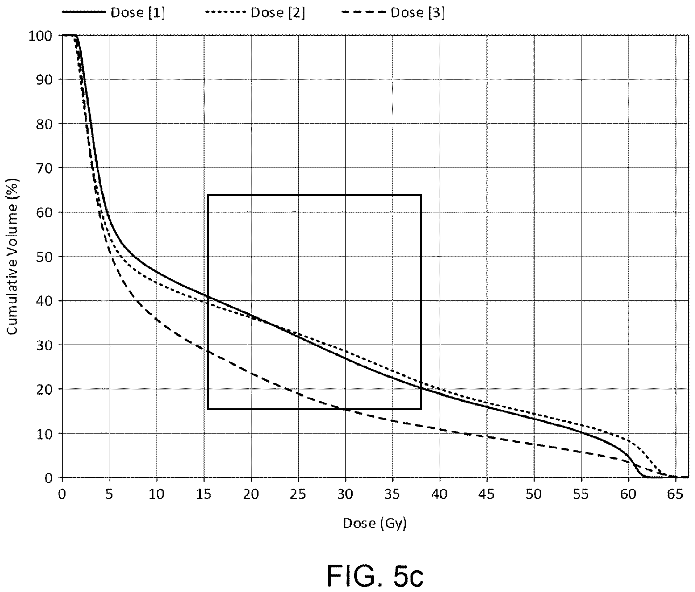

Figure 5a depicts differences in the dose applied to the target, i.e. the tumour. As can be appreciated, the dose received by the tumour is high and nearly identical in each of the plans. Figure 5b compares the doses received by the rectum according to each of the dose distributions. It can be seen that the dose supplied to the rectum is significantly reduced in each of the adapted plans (dashed and unbroken lines) compared to the reference plan (dotted line). Finally, figure 5c compares the doses received by the bladder between the three dose distributions. It can be seen that the dose supplied to the bladder is significantly reduced in the second adapted plan (dashed line) compared to the reference plan (dotted line) and the first adapted plan (unbroken line).

The dose distributions and associated plans depicted in figures 4b and 4c were generated based upon the same reference image used to generate the original 'reference' plan depicted in figure 4a. However, as described elsewhere herein, in some implementations, upon reporting of side effects / symptoms, then the latest available treatment planning image may be used, such as the latest session CBCT image. In this alternative workflow, auto-segmentation techniques may be used to create new contours, and the dose distribution of the new plan is calculated on the new image to confirm it is appropriate. After confirming suitability, this plan may be used for the remainder of the patient's treatment course, whilst frequent monitoring of the patient's side effects is performed to ensure that no other side effects are experienced.

Several benefits and advantages arise from the presently disclosed method(s). In prior art adaptive radiotherapy workflows, treatment plans can be updated to 'adapt' to the patient's anatomical changes, be these changes relate to the tumor shape and size, deformation of OARs, weight loss, and the like. Whilst known workflows are effective and enable a dynamic radiotherapy course to be delivered, they do not allow for treatment to be updated to take into account unexpected side effects and symptoms due to the patient's own radiobiological hypersensitivity. Sometimes, patient side effects and symptoms are not captured through anatomical changes visible in medical images, and patients may not be comfortable reaching out to a clinician if they believe the side effect they are experiencing might just be part of their planned radiotherapy.

Accordingly, by generating a second (e.g. adapted) radiotherapy treatment plan based in part on symptom data, the newly generated plan can be adapted based on patient experience, instead of just observed anatomical changes. Low-grade side effects can be better managed through this process, leading to better management of side effects and symptoms at the individual patient level. In addition, the resulting plan is better tailored to the individual patient and the radiobiological sensitivity of their own organs and tissue.

In addition, by generating / adapting a plan based on the symptoms and side effects the patient is experiencing, the cost burden in managing those side effects through medications, appointments, and tests will be reduced. In addition, the method can be automated and performed offline, reducing the need for online adaptation which would otherwise require significant time and resources on the day of treatment.

In addition, by adapting the plan based on self-reported symptom data, for example provided via a mobile app or the like, the need for ad-hoc in-person meetings between patient and physician is reduced. This is advantageous, as radiotherapy patients can be very sick people who find travel difficult. Reducing the number of in-person meetings between patients and clinicians also has a positive environmental impact by significantly decreasing the need for patient travel. This reduction in travel leads to lower carbon emissions, as fewer trips by car or public transportation journeys are necessary. It also reduces the overall consumption of fossil fuels and diminishes traffic congestion, which further cuts down on air pollution. Additionally, less travel can reduce the demand for infrastructure maintenance and expansion, contributing to a smaller environmental footprint.

Adopting workflows according to the present application support sustainability efforts by minimising the environmental impact associated with frequent travel.

As described herein, in some implementations the newly generated plan(s) are generated based on the latest available imaging, meaning that the resulting adapted plan is not only adapted based on the patient's symptoms but also based on the most up-to-date information available as to the patient's anatomy. In an example, the present adaptive workflow can utilise recent CBCT images, which are more recent representations of a patient's anatomy, in effect creating more anatomically relevant and accurate plans as a by-product of the method.

While the method(s) of the present disclosure have been described in specific ways to aid clarity of understanding, the skilled person will understand that not all of the steps described in relation to a particular method are essential. Also, some of the steps may be performed in a different order. Several variants of the presently disclosed methods exist and some will now be described.

While the application has been described primarily in relation to a multi-criterial optimisation approach based on a wish list comprising a series of ranked objectives, this is not essential and the plan may be adapted based on symptom data in different ways. For example, fluence map optimisation could be performed by a single minimization of a weighted-sum cost function that simultaneously considers all clinical objectives, each with an assigned, optimised weight. In such an implementation, and with reference to blocks 240 and 340 of methods 200 and 300, the method may comprise identifying a term of the cost function associated with the anatomical structure causing the symptom. Generating the adapted radiotherapy plan may comprise adapted the term associated with the anatomical structure, for example increasing its weight so that the term becomes more important during the optimisation process.

In addition, while the application has been described primarily with respect to a wish list containing a set of fixed, immutable constraints and a series of ranked objectives, this needn't be the case. For example, in some implementations, there may be no "fixed" constraints. Instead the entire wish list may be ranked by importance.

In addition, while symptom data has primarily been described as being self-reported symptom data collected e.g.

via a questionnaire presented via a mobile application, this needn't be the case and the symptom data may be collected in different ways. For example, symptom data could come via a discussion with the physician or other hospital staff on the day of treatment, or by observations made by those same hospital staff on the treatment days. In another example, the data could be provided by a wearable or a mobile device. In this example, the symptom data may comprise heart rate data, number of steps, blood glucose levels, and the like. Based on this symptom data, it is possible to extract or infer the existence or severity of at least some categories of symptoms experienced by a radiotherapy patient undergoing treatment according to a radiotherapy plan. For example, glucose monitoring data could indicate a reduction in a patient's appetite or digestive function, which could be associated with a radiation dose applied to the patient's stomach.

In addition, after delivery of the first fraction according to any newly generated plan, a physician may make the decision to continue with utilization of the new plan for the remainder of treatment, revert back to the original plan, or may use the new plan for a set number of fractions, with a monitoring mechanism put in place to monitor patient side effects.

Figure 6 depicts a radiotherapy device 600. The radiotherapy device 600 is suitable for delivering, and configured to deliver, a beam of radiation 610 to a patient during radiotherapy treatment. The device 610 and its constituent components will be described generally for the purpose of providing useful accompanying information for the present application. The device 600 depicted in figure 1 is in accordance with the present disclosure and is suitable for use with the disclosed systems and apparatuses. While the device 600 in figure 6 is an MR-linac, in implementations of the present disclosure the device 600 may be another type of radiotherapy device, for example a linac device with a different imaging capability.

In overview, the radiotherapy device 600 comprises a source of radiation 605 and an imaging apparatus 612. The source of radiation 605 is coupled to a rotatable gantry 61. The device 600 comprises a patient positioning apparatus which comprises a patient positioning surface 614 on which a patient may be positioned. Before treatment, the patient is positioned on the surface 614, and the patient positioning apparatus may be used to position the patient in an appropriate position for the treatment, for example according to a reference image on which the patient's treatment plan is based. During treatment, the source of radiation 605 delivers radiation to the patient according to the patient's treatment plan.

The source of radiation 605 is configured to generate a beam of radiation 610, and in particular a beam of therapeutic radiation. The radiation source 605 is attached to the rotatable gantry 6 so as to rotate with the gantry 6. In this way, the radiation source is rotatable around the patient so that the beam 610 can be applied from different angles around the gantry 61. The source of radiation 605 may comprise a beam generation system comprising a linear accelerator (linac). For such a linac device, the beam generation system may comprise a source of RF energy 602, an electron gun 60, and a waveguide 604.

The source 602 of radiofrequency waves, such as a magnetron, is configured to produce radiofrequency waves. The source 602 of radiofrequency waves is coupled to the waveguide 604, for example via a circulator, and is configured to pulse radiofrequency waves into the waveguide 604. Radiofrequency waves may pass from the source 602 of radiofrequency waves through an RF input window and into an RF input connecting pipe or tube. A source of electrons 60, such as an electron gun, is also coupled to the waveguide 604 and is configured to inject electrons into the waveguide 604. In the electron gun 60, electrons are thermionically emitted from a cathode filament as the filament is heated. The temperature of the filament controls the number of electrons injected. The injection of electrons into the waveguide 604 is synchronised with the pumping of the radiofrequency waves into the waveguide 604. The design and operation of the radiofrequency wave source 602, electron source and the waveguide 604 is such that the radiofrequency waves accelerate the electrons to very high energies as the electrons propagate through the waveguide 604.

The design of the waveguide 604 depends on whether the linac accelerates the electrons using a standing wave or travelling wave, though the waveguide typically comprises a series of cells or cavities, each cavity connected by a hole or 'iris' through which the electron beam may pass. The cavities are coupled in order that a suitable electric field pattern is produced which accelerates electrons propagating through the waveguide 604. As the electrons are accelerated in the waveguide 604, the electron beam path is controlled by a suitable arrangement of steering magnets, or steering coils, which surround the waveguide 604. The arrangement of steering magnets may comprise, for example, two sets of quadrupole magnets.

To ensure that propagation of the electrons is not impeded as the electron beam travels toward the target, the waveguide 604 is evacuated using a vacuum system comprising a vacuum pump or an arrangement of vacuum pumps. The pump system is capable of producing ultra-high vacuum (UHV) conditions in the waveguide 604. The vacuum system also ensures UHV conditions in the electron gun. Electrons can be accelerated to speeds approaching the speed of light in the evacuated waveguide 604.

Once the electrons have been accelerated, they travel toward a heavy metal target which, when impacted by the electrons, generates a beam of high energy photons, forming a radiation beam 610. When the electrons strike the target, X-rays are produced in a variety of directions. The device 600 comprises collimation apparatus 608. The collimation apparatus 608 may comprise a primary collimator. The primary collimator is configured to block X-rays travelling in certain directions and pass only forward travelling X-rays to produce a treatment beam 610. The X-rays may be filtered and may pass through one or more ion chambers for dose measuring. The collimation apparatus 608 may additionally comprise beam shaping apparatus such as a multi-leaf collimator (MLC). The beam can be shaped in various ways by the beam-shaping apparatus. The source of radiation is configured to direct the beam 610 of therapeutic radiation, having been appropriately filtered and shaped by the collimation apparatus 608, toward a patient positioned on the patient support surface 614.

The device 600 comprises an imaging apparatus 612 or 'image acquisition apparatus'. The depicted imaging apparatus 612 is an MR imaging apparatus, though the imaging apparatus may take other forms, for example a cone beam computed tomography (CBCT) apparatus. The MR imaging apparatus 612 is shown in cross-section in the diagram. The imaging apparatus 612 is configured to generate imaging data. The imaging data may comprise images of the patient. The imaging apparatus 612 is therefore configured to obtain images of a patient positioned on the patient support surface 614. The imaging data generated by the imaging apparatus 612 may be used to generate a reference image to enable treatment planning, and/or may be used during the delivery of therapeutic radiation to help guide the beam of radiation 610 or to provide an input into motion management and real-time adaptive radiotherapy techniques.

Figure 6 and its accompanying description herein is provided to give context to the application and to facilitate understanding of the present invention. In addition to the components described in overview above, a radiotherapy device also comprises many other functions, components and subsystems as will be understood by the skilled person.

Figure 7 illustrates a block diagram of one implementation of a radiotherapy system 700. The radiotherapy system 700 comprises a computing system 710 within which a set of instructions, for causing the computing system 710 to perform any one or more of the methods discussed herein, may be executed.

The computing system 710 shall be taken to include any number or collection of machines, e.g. computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 710 includes controller circuitry 711 and a memory 713 (e.g., read-only memory (ROM), flash memory, dynamic random access memory (DRAM) such as synchronous DRAM (SDRAM) or Rambus DRAM (RDRAM), etc.). The memory 713 may comprise a static memory (e.g., flash memory, static random access memory (SRAM), etc.), and/or a secondary memory (e.g., a data storage device), which communicate with each other via a bus (not shown).

Controller circuitry 711 represents one or more general-purpose processors such as a microprocessor, central processing unit, accelerated processing units, or the like. More particularly, the controller circuitry 711 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 711 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 711 is configured to execute the processing logic for performing the operations and steps discussed herein.

The computing system 710 may further include a network interface circuitry 715. The computing system 710 may be communicatively coupled to an input device 720 and/or an output device 730, via input/output circuitry 717. In some implementations, the input device 720 and/or the output device 730 may be elements of the computing system 710. The input device 720 may include an alphanumeric input device (e.g., a keyboard or touchscreen), a cursor control device (e.g., a mouse or touchscreen), an audio device such as a microphone, and/or a haptic input device. The output device 730 may include an audio device such as a speaker, a video display unit (e.g., a liquid crystal display (LCD) or a cathode ray tube (CRT)), and/or a haptic output device. In some implementations, the input device 720 and the output device 730 may be provided as a single device, or as separate devices.

In some implementations, computing system 710 includes training circuitry 718. The training circuitry 718 is configured to train a classifier. The classifier may be configured to map symptom data to an associated anatomical structure. For example, training circuitry 718 may train a model for performing a method of associating symptom data with associated anatomical structure; for example the anatomical structure which, due to receipt of radiation during the application of a radiotherapy treatment plan, is responsible for the symptom represented by the symptom data. The model may comprise a deep neural network (DNN), such as a convolutional neural network (CNN) and/or recurrent neural network (RNN). Training circuitry 718 may be configured to execute instructions to train a model that can be used to determine, based on the symptom data, an anatomical structure associated with the symptom, as described with reference to block 220 of figure 2. Training circuitry 718 may be configured to access training data and/or testing data from memory 713 or from a remote data source, for example via network interface circuitry 715. In some examples, training data and/or testing data may be obtained from an external component, such as image acquisition device 740 and/or treatment device 750. The model may be trained using a training data set comprising, for example, a database of self-reported symptom data from patients. The training data may contain, for each patient's self-reported symptom data, an association made by a clinician of which anatomical region is associated with, e.g. responsible for, the patient's self-reported symptoms. Such data can be collated by asking clinicians to review many different patient's self-reported symptoms and making an assessment of whether the symptoms are associated with a particular anatomical feature having received radiation according to the patient's treatment plan. The training data may therefore comprise previous, historic symptom data, an associated determination of whether the symptom(s) are associated with the treatment plan, and if so which anatomical feature is associated with the symptoms. In some implementations, training circuitry 718 may be used to update, verify and/or maintain the model.

In some implementations, the computing system 710 may comprise image processing circuitry 719. Image processing circuitry 719 may be configured to process image data 780 (e.g. images, or imaging data), such as medical images obtained from one or more imaging data sources, a treatment device 750 and/or an image acquisition device 740. Image processing circuitry 719 may be configured to process, or pre-process, image data. For example, image processing circuitry 719 may convert received image data into a particular format, size, resolution or the like. In some implementations, image processing circuitry 719 may be combined with controller circuitry 711.

In some implementations, the radiotherapy system 700 may further comprise an image acquisition device 740 and/or a treatment device 750, such as those disclosed herein in the example of Fig. 6. The image acquisition device 740 and the treatment device 750 may be provided as a single device. In some implementations, treatment device 750 is configured to perform imaging, for example in addition to providing treatment and/or during treatment.

Image acquisition device 740 may be configured to output image data 780, which may be accessed by computing system 710. Treatment device 750 may be configured to output treatment data 760, which may be accessed by computing system 710.

Computing system 710 may be configured to access or obtain treatment data 760, planning data 770 and/or image data 780. Treatment data 760 may be obtained from an internal data source (e.g. from memory 713) or from an external data source, such as treatment device 750 or an external database. Planning data 770 may be obtained from memory 713 and/or from an external source, such as a planning database. Planning data 770 may comprise information obtained from one or more of the image acquisition device 740 and the treatment device 750.

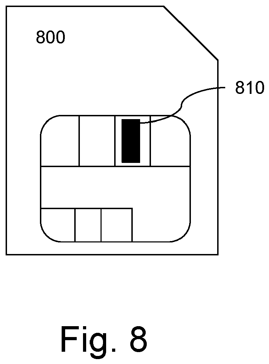

The various methods described above may be implemented by a computer program. The computer program may include computer code (e.g. instructions) 810 arranged to instruct a computer to perform the functions of one or more of the various methods described above. The steps of the methods described above may be performed in any suitable order. For example, step 360 of method 3 may be performed first in method 300, where a patient is prompted to report symptoms of their current radiotherapy treatment plan. The computer program and/or the code 810 for performing such methods may be provided to an apparatus, such as a computer, on one or more computer readable media or, more generally, a computer program product 800, depicted in Figure 8. The computer readable media may be transitory or non-transitory. The one or more computer readable media 800 could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions 810 may also reside, completely or at least partially, within the memory 713 and/or within the controller circuitry 711 during execution thereof by the computing system 710, the memory 713 and the controller circuitry 711 also constituting computer-readable storage media.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

**EP 4 751 769 A1**

Unless specifically stated otherwise, as apparent from the following discussion, it is appreciated that throughout the description, discussions utilizing terms such as " receiving", "determining", "comparing ", "obtaining", "generating," "identifying," or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

**Claims**

1. A computer-implemented method for adaptive radiotherapy, comprising:

   receiving symptom data, the symptom data being indicative of the existence and/or severity of a symptom experienced by a radiotherapy patient undergoing treatment according to a first radiotherapy treatment plan;
   obtaining first treatment plan data associated with the first radiotherapy treatment plan; and
   generating a second radiotherapy treatment plan based on the obtained first treatment plan data and the symptom data.

2. The method further comprising determining, based on the symptom data, an anatomical structure associated with the symptom.

3. The method of claim 2, wherein the second radiotherapy treatment plan defines a reduced dose to the anatomical structure compared with a first planned dose defined by the first treatment plan.

4. The method of claim 2 or claim 3, wherein the first treatment plan data comprises a radiotherapy planning image of the patient, wherein the radiotherapy planning image depicts a target region and the anatomical structure.

5. The method of any of claims 2 to 4, wherein the first treatment plan data comprises a first set of objectives used to guide a first optimisation process during generation of the first treatment plan.

6. The method of claim 5, wherein each of the first set of objectives has an ascribed priority and the first set of objectives is ranked in order of importance according to the ascribed priorities to provide a guide for the first optimisation process.

7. The method of claim 6, wherein the first radiotherapy treatment plan has been generated, at least in part, by sequentially optimising for the first set of objectives in turn, according to their ascribed priorities.

8. The method of any of claims 5 to 7, wherein generating the second radiotherapy treatment plan comprises: adapting the first set of objectives to form an adapted set of objectives; and performing a second optimisation process based on the adapted set of objectives.

9. The method of claim 8, wherein adapting the first set of objectives comprises: adding a new objective to the first set of objectives, the new objective relating to the anatomical structure.

10. The method of any of claim 8 or claim 9, wherein at least one of the objectives of the first set of objectives relates to the anatomical structure; and
    wherein adapting the first set of objectives comprises adapting the at least one objective relating to the anatomical structure to form the adapted set of objectives.

11. The method of any of claims 8 to 10, wherein each objective of the adapted set of objectives has an ascribed priority and the adapted set of objectives is ranked in order of importance according to the ascribed priorities to provide a guide for the second optimisation process, wherein the at least one objective associated with the anatomical structure has an increased priority in the adapted set of objectives in comparison with its priority in the first set of objectives.

12. The method of any of claims 8 to 11, wherein the second radiotherapy plan is a plurality of radiotherapy treatment plans, each plan of the plurality of radiotherapy treatment plans comprising a different trade-off between the objectives of the adapted set of objectives.

13. A computer readable medium comprising instructions which, when executed by one or more processors, cause the one or more processors to implement the method according to any preceding claim.

14. A radiotherapy system comprising one or more processors configured to perform the method of any of claims 1 to 12.

15. The radiotherapy system of claim 14, further comprising a source of therapeutic radiation configured to deliver the generated second radiotherapy treatment plan.

FIG. 1A

150

Fluence Map
Optimisation — 151

Determine
configuration — 153

FIG. 1B

200

**210** Receive symptom data, the symptom data being indicative of the existence or severity of a symptom experienced by a radiotherapy patient undergoing treatment according to a first radiotherapy plan

**220** Determine, based on the symptom data, an anatomical structure associated with the symptom

**230** Obtain first treatment plan data associated with the first radiotherapy treatment plan

**240** Generate a second radiotherapy treatment plan based on the obtained first treatment plan data and the symptom data.

FIG. 2

300

**310 Receive symptom data, the symptom data being indicative of the existence or severity of a symptom experienced by a radiotherapy patient undergoing treatment according to a radiotherapy plan**

**320 Determine, based on the symptom data, an anatomical structure associated with the symptom**

**360 Prompt the patient to report symptoms experienced following delivery of radiation according to the adapted radiotherapy treatment plan**

**330 Obtain treatment plan data associated with the radiotherapy treatment plan**

**350 Deliver radiation to the patient according to the adapted radiotherapy treatment plan**

**340 Generate an adapted radiotherapy treatment plan based on the obtained treatment plan data and the symptom data.**

FIG. 3

FIG. 4a

FIG. 4b

FIG. 4c

FIG. 5a

FIG. 5b

FIG. 5c

Fig. 6

Fig. 7

Fig. 8

**EUROPEAN SEARCH REPORT**

Application Number

EP 24 21 5917

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/136455 A1 (BHARAT SHYAM [US] ET AL) 19 May 2016 (2016-05-19) | 1-5, 8-10, 12-15 | INV. A61N5/10 |
| Y | * paragraphs [0014] - [0032] * <br> * figures 1, 4 * | 6,7,11 | |
| X | US 2012/004492 A1 (WEIBRECHT MARTIN [DE] ET AL) 5 January 2012 (2012-01-05) <br><br> * paragraphs [0007] - [0016], [0025] - [0031], [0040] * | 1-5, 8-10, 12-15 | |
| X | KR 2021 0132947 A (INDUSTRY ACADEMIC COOPERATION FOUNDATION OF YEUNGNAM UNIV) 5 November 2021 (2021-11-05) <br> * paragraphs [0005] - [0019], [0031] - [0046] * | 1-5, 8-10, 12-15 | |
| X | US 2012/136194 A1 (ZHANG XIAODONG [US] ET AL) 31 May 2012 (2012-05-31) <br><br> * paragraphs [0011] - [0025], [0110] - [0127], [0178] - [0203] * | 1-5, 8-10, 12-15 | TECHNICAL FIELDS SEARCHED (IPC) <br><br> A61N |
| Y | US 2024/374925 A1 (VOET PETER [US] ET AL) 14 November 2024 (2024-11-14) <br> * paragraphs [0223] - [0233] * | 6,7,11 | |

The present search report has been drawn up for all claims

| Place of search | Date of completion of the search | Examiner |
|---|---|---|
| Munich | 24 April 2025 | Lohmann, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 5917

This annex lists the patent family members relating to the patent documents cited in the above-mentioned European search report.
The members are as contained in the European Patent Office EDP file on
The European Patent Office is in no way liable for these particulars which are merely given for the purpose of information.

24-04-2025

| Patent document cited in search report | Publication date | Patent family member(s) | Publication date |
|---|---|---|---|
| US 2016136455 A1 | 19-05-2016 | CN 105358218 A | 24-02-2016 |
| | | EP 3013419 A1 | 04-05-2016 |
| | | JP 6259078 B2 | 10-01-2018 |
| | | JP 2016529953 A | 29-09-2016 |
| | | US 2016136455 A1 | 19-05-2016 |
| | | WO 2014207663 A1 | 31-12-2014 |
| US 2012004492 A1 | 05-01-2012 | BR PI1006689 A2 | 12-04-2016 |
| | | CN 102365111 A | 29-02-2012 |
| | | EP 2411094 A1 | 01-02-2012 |
| | | JP 5718892 B2 | 13-05-2015 |
| | | JP 2012521792 A | 20-09-2012 |
| | | RU 2011143355 A | 10-05-2013 |
| | | US 2012004492 A1 | 05-01-2012 |
| | | WO 2010109357 A1 | 30-09-2010 |
| KR 20210132947 A | 05-11-2021 | NONE | |
| US 2012136194 A1 | 31-05-2012 | CN 103282967 A | 04-09-2013 |
| | | EP 2605828 A2 | 26-06-2013 |
| | | US 2012136194 A1 | 31-05-2012 |
| | | WO 2012024448 A2 | 23-02-2012 |
| US 2024374925 A1 | 14-11-2024 | CN 118925083 A | 12-11-2024 |
| | | EP 4487906 A1 | 08-01-2025 |
| | | US 2024374925 A1 | 14-11-2024 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 38100 A1

**Non-patent literature cited in the description**

- **BREEDVELD S ; STORCHI PR ; VOET PW ; HEIJMEN BJ**. iCycle: Integrated, multicriterial beam angle, and profile optimization for generation of coplanar and noncoplanar IMRT plans. *Med Phys*, February 2012, vol. 39 (2), 951-3

- **BIJMAN R ; SHARFO AW ; ROSSI L ; BREEDVELD S ; HEIJMEN B.** Pre-clinical validation of a novel system for fully-automated treatment planning.. *Radiother Oncol.*, 10 March 2021, vol. 158, 253-21